# EUROPEAN PATENT SPECIFICATION

(11) **EP 2 505 835 B1**
(45) Date of publication and mention of the grant of the patent: **30.03.2016**
(21) Application number: 10833179.4
(22) Date of filing: 22.11.2010
(51) Int. Cl.: F04B 3/00, A61M 1/00, A61M 1/14, F04B 53/02, F04B 13/02

(54) **RECIPROCATION PUMP AND DIALYSIS DEVICE COMPRISING SAME**
HUBKOLBENPUMPE UND DIALYSEVORRICHTUNG DAMIT
POMPE ALTERNATIVE ET DISPOSITIF DE DIALYSE COMPRENANT CETTE POMPE

(30) Priority: 24.11.2009 JP 2009266395
(43) Date of publication of application: 03.10.2012
(73) Proprietor: Nikkiso Company Limited, Tokyo 150-8677 (JP)
(72) Inventor: SUZUKI, Hiroaki, Makinohara-shi Shizuoka 421-0496 (JP)
(74) Representative: von Hirschhausen, Helge
(86) International application number: PCT/JP2010/070826
(87) International publication number: WO 2011/065333

(56) References cited:
- WO-A1-93/22556
- DE-A1- 19 743 917
- FR-A1- 2 335 708
- JP-A- 1 008 367
- JP-A- 10 299 642
- JP-A- 50 024 675
- JP-A- 2003 284 772
- JP-A- 2003 284 772
- US-A1- 2004 057 853

## Description

### Field of the invention

The present invention relates to a reciprocation pump for supplying a liquid from the source to an objective apparatus with the supply liquid-side pumping chamber and discharging to the outside a waste liquid from the objective apparatus with the waste liquid-side pumping chamber, and to a dialysis apparatus equipped with the reciprocation pump.

### Description of Background Art

In general, a blood purification apparatus such as a dialyzer is used in hemodialysis treatment and a dialysate supplying line for supplying the dialysate as well as a dialysate discharging line for discharging the waste dialysate are connected to the blood purification apparatus. These dialysate supplying line and dialysate discharging line are extended from a dialysis apparatus main body and connected to the blood purification apparatus to supply the dialysate to the blood purification apparatus and to discharge the waste dialysate from the blood purification apparatus to the outside.

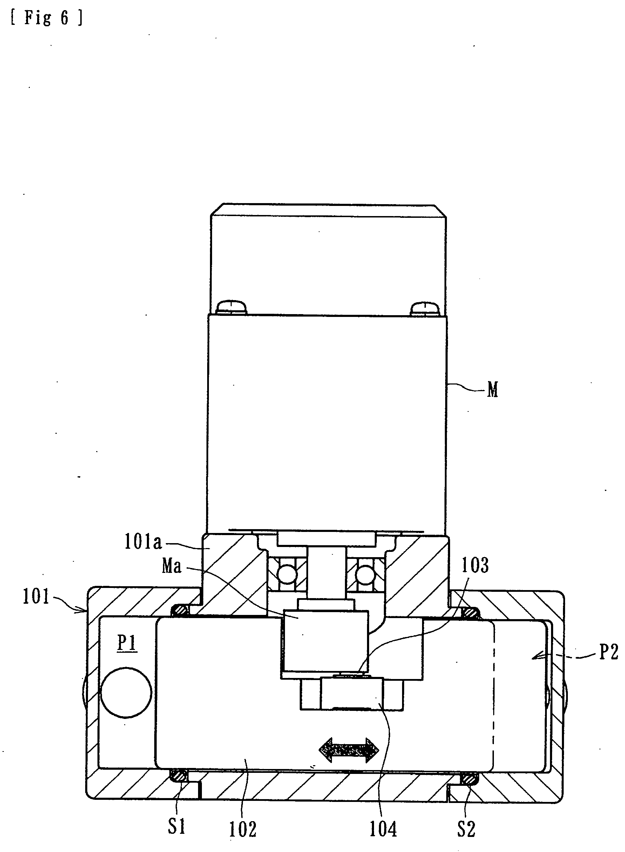

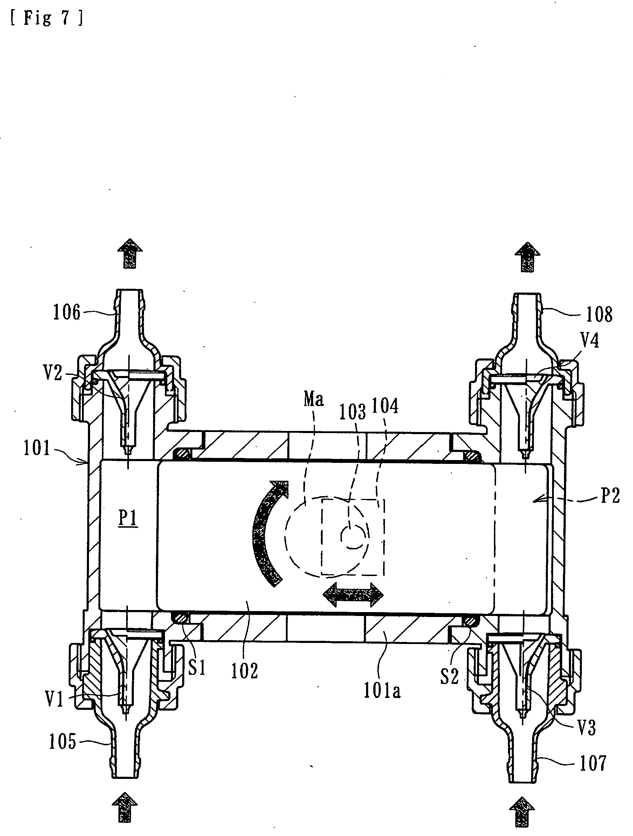

The reciprocation pump (duplex pump) is connected to the dialysis apparatus main body across the dialysate supplying line and the dialysate discharging line. As shown in Figs. 6 and 7, the reciprocation pump comprises a casing 101 for reciprocatably accommodating a plunger 102, a supply liquid-side pumping chamber P1 and a waste liquid-side pumping chamber P2 formed by a plunger 102 within the casing 101, supply liquid-side ports 105, 106 communicating with the supply liquid-side pumping chamber P1, waste liquid-side ports 107, 108 communicating with the waste liquid-side pumping chamber P2, and a motor M for reciprocating the plunger 102.

The supply liquid-side ports 105, 106 are provided with check valves V1, V2 respectively and the waste liquid-side ports 107, 108 are also provided with check valves V3, V4 respectively. The reciprocation pump is so structured that the supply liquid-side pumping chamber P1 sucks the liquid (dialysate) from its source through the supply liquid-side port 105 and supplies it to a blood purification apparatus (not shown in Figs. 6 and 7) through the supply liquid-side port 106 and the waste liquid-side pumping chamber P2 receives the waste liquid (waste dialysate) from the blood purification apparatus through the waste liquid-side port 107 and discharges it the outside through the waste liquid-side port 108.

An eccentric cam 103 is mounted on an output shaft Ma of the motor M and a block 104 reciprocated by the eccentric cam 103 is mounted on the plunger 102. With driving the motor M, the plunger 102 can be reciprocated via the block 104 and the supply and discharge of the dialysate can be performed as described above. Such a reciprocation pump is disclosed e.g. in Patent Document 1 below.

### Document of Prior Art

### Patent Document

Patent Document 1: Japanese Laid-open Patent Publication No. 2003-284772

### Disclosure of the Invention

### Problems to be solved by the Invention

However, in such a reciprocation pump of the prior art shown in Figs. 6 and 7, since it requires a motor casing 101a for mounting the motor M on the pump casing 101 as well as sealing members S1, S2 interposed between the motor casing 101a and the pump casing 101, it is believed that the dialysate to be supplied to the blood purification apparatus and the waste dialysate would be leaked to the outside of the reciprocation pump due to aged degradation of the sealing members S1, S2. Such a problem is also found in reciprocal pumps in other fields for supplying liquids to objective apparatus other than the blood purification apparatus.

It is, therefore, an object of the present invention to provide a reciprocation pump which can prevent a liquid, to be supplied to an objective apparatus, and a waste liquid, to be discharged from the objective apparatus, from leaking outside of the reciprocation pump as well as a dialysis apparatus equipped with the reciprocation pump.

### Means for solving problems

For achieving the object mentioned above, there is provided, according to the present invention of claim 1, a reciprocation pump comprising a supply liquid-side pumping chamber for supplying a liquid from a source to an objective apparatus; a waste liquid-side pumping chamber for discharging a waste liquid discharged from the objective apparatus to the outside; a reciprocation means being able to reciprocate between the supply liquid-side pumping chamber and the waste liquid-side pumping chamber, the reciprocating motion of the reciprocation means performing the suction and discharge of the liquid to and from the supply liquid-side pumping chamber as well as the suction and discharge of the waste liquid to and from the waste liquid-side pumping chamber; and a driving source for driving the reciprocation means; the reciprocation pump adapted to supply the liquid from the source to the objective apparatus with the supply liquid-side pumping chamber and to discharge the waste liquid from the objective apparatus to the outside with the waste liquid-side pumping chamber and characterized in further comprising a supply liquid-side sub-pumping chamber arranged adjacent to the supply liquid-side pumping chamber at the external side thereof; a waste liquid-side sub-pumping chamber arranged adjacent to the waste liquid-side pumping chamber at the external side thereof; a rod extending through the supply liquid-side sub-pumping chamber, the supply liquid-side pumping chamber, the waste liquid-side pumping chamber and the waste liquid-side sub-pumping chamber and being connected to the reciprocation means and the driving source to transmit a driving force of the driving source to the reciprocation means; and sealing means arranged respectively in walls through which the rod extends for partitioning the supply liquid-side pumping chamber and the supply liquid-side sub-pumping chamber as well as the waste liquid-side pumping chamber and the waste liquid-side sub-pumping chamber.

It is preferable as defined in claim 2 that the reciprocation pump further comprises diaphragms respectively mounted on the distal end and the proximal end of the rod to form the supply liquid-side sub-pumping chamber and the waste liquid-side sub-pumping chamber respectively.

It is preferable as defined in claim 3 that the liquid is supplied to the objective apparatus through both the supply liquid-side pumping chamber and the supply liquid-side sub-pumping chamber, and that waste liquid is discharged to the outside through both the waste liquid-side pumping chamber and the waste liquid-side sub-pumping chamber.

It is also preferable as defined in claim 4 that the reciprocation means comprises a plunger formed on the rod to form the supply liquid-side pumping chamber and the waste liquid-side pumping chamber, and that in a central sealing means is arranged between the supply liquid-side pumping chamber and the waste liquid-side pumping chamber at a substantially reciprocating center of the plunger to seal the supply liquid-side pumping chamber and the waste liquid-side pumping chamber from each other.

It is preferable as defined in claim 5 that the reciprocation means comprises a diaphragm mounted on the rod to form the supply liquid-side pumping chamber and the waste liquid-side pumping chamber respectively.

It is also preferable as defined in claim 6 that the objective apparatus is a blood purification apparatus and the liquid is dialysate.

It is preferable as defined in claim 7 that the present invention also provide a dialysis apparatus equipped with the reciprocation pump of claim 6.

### Effects of the invention

According to the present invention of claim 1, since the supply liquid-side sub-pumping chamber and the waste liquid-side sub-pumping chamber are arranged at the external sides respectively of the supply liquid-side pumping chamber and the waste liquid-side pumping chamber, and since the sealing means are arranged respectively in walls for partitioning the supply liquid-side pumping chamber and the supply liquid-side sub-pumping chamber as well as the waste liquid-side pumping chamber and the waste liquid-side sub-pumping chamber, this makes it possible to keep the constancy of volumes of the supply liquid-side pumping chamber and the waste liquid-side pumping chamber. In addition, this makes it possible to prevent the liquid (dialysate) from being leaked to the outside since the liquid will be flowed into the sub-pumping chambers respectively of the supply liquid-side pumping chamber and the waste liquid-side pumping chamber even if any leakage of the liquid would be caused due to aged degradation of the sealing means.

According to the present invention of claim 2, since the reciprocation pump further comprises diaphragms respectively mounted on the distal end and the proximal end of the rod to form the supply liquid-side sub-pumping chamber and the waste liquid-side sub-pumping chamber respectively, it is possible to eliminate necessity of provision of separate sealing means at the mounting portions of the diaphragms.

According to the present invention of claim 3, since the liquid is supplied to the objective apparatus through both the supply liquid-side pumping chamber and the supply liquid-side sub-pumping chamber, and waste liquid is discharged to the outside through both the waste liquid-side pumping chamber and the waste liquid-side sub-pumping chamber, it is possible to supply leaked liquid to the objective apparatus through the supply liquid-side sub-pumping chamber if the liquid would be leaked from the supply liquid-side pumping chamber to the supply liquid-side sub-pumping chamber due to aged degradation of the sealing means and similarly possible to discharge the leaked waste liquid from the waste liquid-side sub-pumping chamber to the outside if the waste liquid would be leaked from the waste liquid-side pumping chamber to the waste liquid-side sub-pumping chamber.

According to the present invention of claim 4, since the central sealing means is arranged between the supply liquid-side pumping chamber and the waste liquid-side pumping chamber at a substantially reciprocating center of the plunger to seal the supply liquid-side pumping chamber and the waste liquid-side pumping chamber from each other, the leaked liquid can flow from the supply liquid-side pumping chamber to the waste liquid-side pumping chamber or the leaked waste liquid can flow from the waste liquid-side pumping chamber to the supply liquid-side pumping chamber, and thus it is possible to prevent the leaked liquid and waste liquid from being leaked to the outside of the reciprocation pump.

According to the present invention of claim 5, since the reciprocation means comprises a diaphragm mounted on the rod to form the supply liquid-side pumping chamber and the waste liquid-side pumping chamber, it is possible to eliminate any sealing means for sealingly form the supply liquid-side pumping chamber and the waste liquid-side pumping chamber as compared with a case using plunger for this purpose.

According to the present invention of claim 6, since the objective apparatus is a blood purification apparatus and the liquid is dialysate, it is possible to prevent the dialysate or the waste dialysate from being leaked to the outside of the reciprocation pump.

According to the present invention of claim 7, it is possible to provide a dialysis apparatus having superior effects of the reciprocation pump defined in claim 6.

### Brief Description of the Drawings

[Fig.1] A longitudinal-section view of a reciprocation pump of a preferable embodiment of the present invention;
[Fig.2] Across-sectional view taken along a line II-II of Fig. 1;
[Fig.3] Across-sectional view taken along a line III-III of Fig. 1;
[Fig.4] A schematic view showing a connection between the reciprocation pump and a blood purification apparatus;
[Fig.5] A longitudinal-section view of a reciprocation pump of another embodiment of the present invention;
[Fig.6] A plan view of longitudinal-section of a reciprocation pump of the prior art; and
[Fig.7] A side elevation view of longitudinal-section of a reciprocation pump of the prior art.

### A Preferable mode for carrying out the Invention

The preferable embodiments of the present invention will be described with reference to the accompanied drawings.

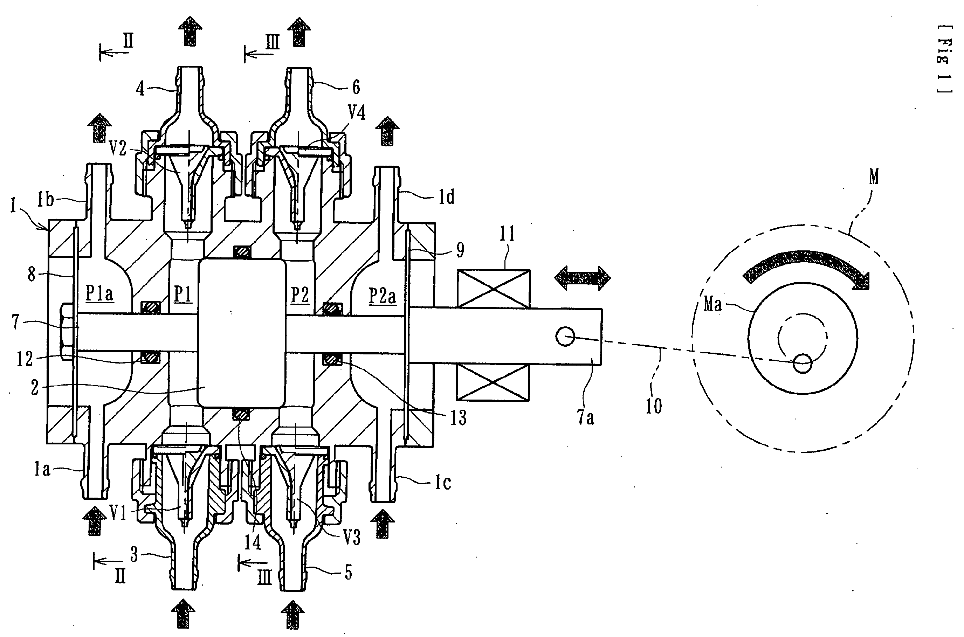

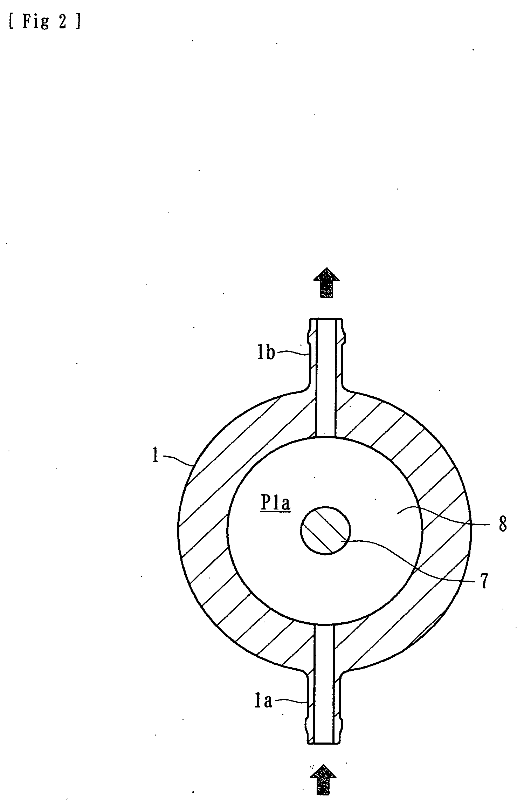

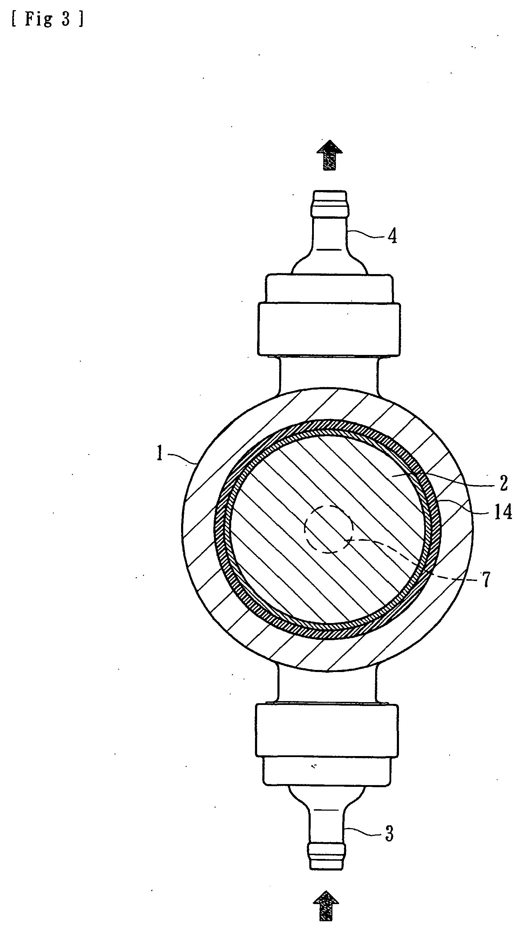

The reciprocation pump of one preferable embodiment of the present invention can be applied to the hemodialysis apparatus and comprises a so-called duplex pump for supplying a liquid to an objective apparatus (e.g. blood purification apparatus) with a supply liquid-side pumping chamber and discharging a waste liquid from the objective apparatus to a waste liquid-side pumping chamber. As shown in Figs. 1~3, the reciprocation pump of the present invention mainly comprises a casing 1 in which a supply liquid-side pumping chamber P1 and a waste liquid-side pumping chamber P2 are formed, a plunger 2 acting as a reciprocation means, a supply liquid-side sub-pumping chamber P1a formed by a diaphragm 8, a waste liquid-side sub-pumping chamber P2a formed by a diaphragm 9, a rod 7, a motor M acting as a driving source, sealing means 12, 13, and a central sealing means 14.

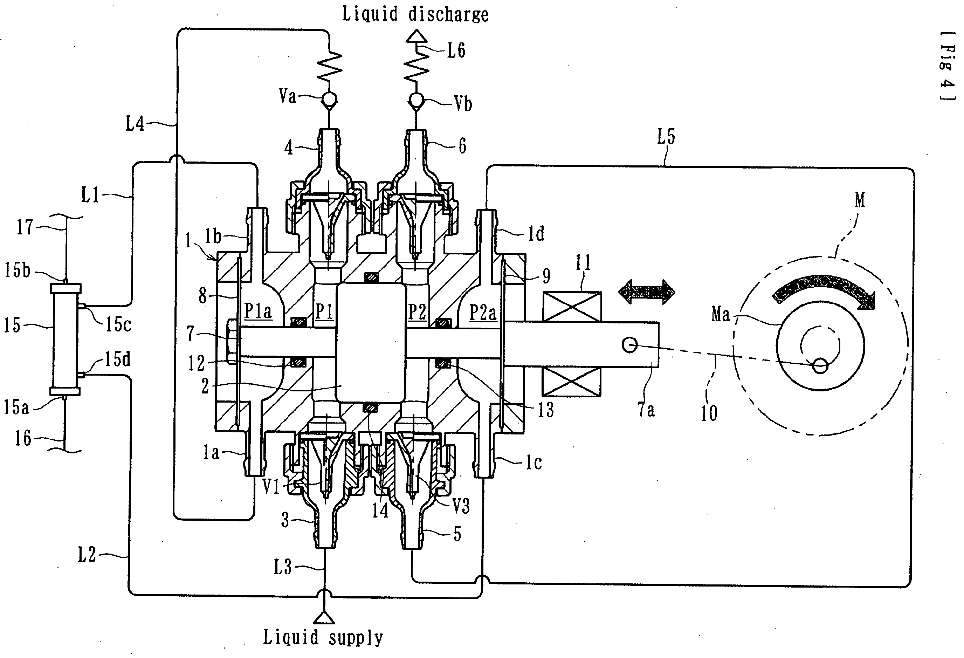

As shown in Fig. 4, the reciprocation pump is connected to a blood purification apparatus 16 acting as the objective apparatus acting. The blood purification apparatus 15 is formed with a blood inlet port 15a, a blood outlet port 15b, dialysate inlet port 15c, and dialysate outlet port 15d. An arterial blood circuit 16 is connected to the blood inlet port 15a and a venous blood circuit 17 is connected to the blood outlet port 15b. In addition, a dialysate supplying line L1 extending from the reciprocation pump is connected to a dialysate inlet port 15c and a dialysate discharging line L2 extending from the reciprocation pump is connected to a dialysate outlet port 15d.

The arterial blood circuit 16 and the venous blood circuit 17 form a blood circuit for extracorporeally circulate a blood of patient and are respectively equipped with an arterial puncture needle and a venous puncture needle (not shown). The blood purification apparatus 15 contains therein an enormous number of hollow fiber membranes (blood purification membranes). The inside of each hollow fiber membrane communicates with the blood inlet port 15a and the blood outlet port 15b and forms a blood flow route. On the other hand, a space between the outer circumferetial surface of each hollow fiber membrane and the inner circumferential surface of the casing of the blood purification apparatus 15 communicates with the dialysate inlet port 15c and the dialysate outlet port 15d to form the dialysate flow route for passing the dialysate (liquid) supplied by the reciprocation pump. Since the hollow fiber membrane is formed with an enormous number of micro pores, unnecessary materials (blood waste materials) can be removed by dialysate via the hollow fiber membranes.

The casing 1 of the reciprocation pump of the present invention is formed of metallic parts or molded hard plastic parts and formed therein the supply liquid-side pumping chamber P1, the waste liquid-side pumping chamber P2, the supply liquid-side sub-pumping chamber P1a, and the waste liquid-side sub-pumping chamber P2a. Projected connection ports 1a, 1b are formed on the casing 1 at the bottom and top of the supply liquid-side sub-pumping chamber P1a and appropriate flow routes (e.g. flexible tubes) can be connected thereto. Similarly, projected connection ports 1c, 1d are formed on the casing 1 at the bottom and top of the waste liquid-side sub-pumping chamber P2a and appropriate flow routes (e.g. flexible tubes) can be connected thereto.

The supply liquid-side pumping chamber P1 is intended to supply the dialysate to the blood purification apparatus 15 (Fig. 4) and a supply liquid-side inlet port 3 and a supply liquid-side outlet port 4 are arranged at the bottom and top of the supply liquid-side pumping chamber P1. Reference characters V1, V2 denote check valves which are mounted respectively on the supply liquid-side inlet port 3 and the supply liquid-side outlet port 4 and act to permit flow of the dialysate from the bottom to the top and to prevent flow of the dialysate from the top to the bottom of the supply liquid-side pumping chamber P1.

The waste liquid-side pumping chamber P2 is intended to discharge a waste liquid (waste dialysate) to the outside of the reciprocation pump and a waste liquid-side inlet port 5 and a waste liquid-side outlet port 6 are arranged at the bottom and top of the waste liquid-side pumping chamber P2. Reference characters V3, V4 denote check valves which are mounted respectively on the waste liquid-side inlet port 5 and the waste liquid-side outlet port 6 and act to permit flow of the waste liquid from the bottom to the top and to prevent flow of the waste liquid from the top to the bottom of the waste liquid-side pumping chamber P2.

The plunger 2 can be reciprocated between the supply liquid-side pumping chamber P1 and the waste liquid-side pumping chamber P2 by the motor M as a driving source in order to perform the suction and discharge of the dialysate to and from the supply liquid-side pumping chamber P1 and the suction and discharge of the waste dialysate to and from the waste liquid-side pumping chamber P2. That is, the suction of the dialysate from its source and the supply of the dialysate to the blood purification apparatus 15 are repeatingly performed in the supply liquid-side pumping chamber P1 as well as the suction of the waste dialysate from the blood purification apparatus 15 and the discharge of the waste dialysate to the outside of the reciprocation pump are repeatingly performed in the waste liquid-side pumping chamber P2 are simultaneously and repeatingly performed in the waste liquid-side pumping chamber P2 during reciprocal motion of the plunger 2.

The supply liquid-side sub-pumping chamber P1a is arranged adjacent to the supply liquid-side pumping chamber P1 at the external side (left-side) thereof and formed by the diaphragm 8 mounted on the rod 7 and is adapted to perform the suction and discharge of the dialysate via the connection ports 1a, 1b. The diaphragm 8 is secured to the casing 1 with its peripheral edge being fitted into the casing via a sealed manner and adapted to be displaced by the rod 7 to vary the volume of the supply liquid-side sub-pumping chamber P1a.

The waste liquid-side sub-pumping chamber P2a is arranged adjacent to the waste liquid-side pumping chamber P2 at the external side (right-side) thereof and formed by the diaphragm 9 mounted on the rod 7 and is adapted to perform the suction and discharge of the waste dialysate via the connection ports 1c, 1d. The diaphragm 9 is secured to the casing 1 with its peripheral edge being fitted into the casing via a sealed manner and adapted to be displaced by the rod 7 to vary the volume of the waste liquid-side sub-pumping chamber P2a.

The rod 7 extends through the supply liquid-side sub-pumping chamber P1a, the supply liquid-side pumping chamber P1, the waste liquid-side pumping chamber P2 and the waste liquid-side sub-pumping chamber P2a and is connected to the plunger 2 (reciprocation means) and the motor M (driving source) to transmit a driving force of the motor M to the plunger 2 and to reciprocate the plunger 2. More particularly, diaphragms 8, 9 are mounted respectively on the distal end and the proximal end of the rod 7 and the plunger 2 is also mounted on the rod 7 at a substantially central portion between the diaphragms 8, 9.

The supply liquid-side sub-pumping chamber P1a can be communicated with the supply liquid-side pumping chamber P1 so that it permits the dialysate to be flowed therethrough. The diaphragm 8 is interlocked with the plunger 2 and varies the volume of the supply liquid-side sub-pumping chamber P1a reversely to the volume variation of the supply liquid-side pumping chamber P1 (i.e. the volume of the supply liquid-side sub-pumping chamber P1a is reduced in accordance with increase of the volume of the supply liquid-side pumping chamber P1, and on the contrary the volume of the supply liquid-side sub-pumping chamber P1a is increased in accordance with reduction of the volume of the supply liquid-side pumping chamber P1). Similarly, the waste liquid-side sub-pumping chamber P2a can be communicated with the waste liquid-side pumping chamber P2 so that it permits the waste dialysate to be flowed therethrough. The diaphragm 9 is interlocked with the plunger 2 and varies the volume of the waste liquid-side sub-pumping chamber P2a reversely to the volume variation of the waste liquid-side pumping chamber P2 (i.e. the volume of the waste liquid-side sub-pumping chamber P2a is reduced in accordance with increase of the volume of the waste liquid-side pumping chamber P2, and on the contrary the volume of the waste liquid-side sub-pumping chamber P2a is increased in accordance with reduction of the volume of the waste liquid-side pumping chamber P2).

As shown in Fig. 1, a crosshead 7a is mounted on the proximal end of the rod 7 and received through a crosshead cylinder 11 for guiding the reciprocal motion of the rod 7. An output shaft Ma of the motor M is connected to the crosshead 7a via a connecting rod 10 and thus the rod 7 can be reciprocated by the rotation of the motor M via the connecting rod 10.

Sealing means 12, 13 comprise sealing parts in common use such as O-rings arranged respectively in walls through which the rod 7 extends for partitioning the supply liquid-side pumping chamber P1 and the supply liquid-side sub-pumping chamber P1a as well as the waste liquid-side pumping chamber P2 and the waste liquid-side sub-pumping chamber P2a. The sealing means 12, 13 allow the reciprocal sliding motion of the rod 7 and prevent leakage of the liquid (dialysate) between the supply liquid-side pumping chamber P1 and the supply liquid-side sub-pumping chamber P1a as well as the waste liquid-side pumping chamber P2 and the waste liquid-side sub-pumping chamber P2a.

A central sealing means 14 also comprises a sealing part in common use such as an O-ring arranged between the supply liquid-side pumping chamber P1 and the waste liquid-side pumping chamber P2 at a substantially reciprocating center of the plunger 2 to seal the supply liquid-side pumping chamber P1 and the waste liquid-side pumping chamber P2 from each other. That is, the supply liquid-side pumping chamber P1 is sealed by the sealing means 12 and the central sealing means 14 and the waste liquid-side pumping chamber P2 is sealed by the sealing means 13 and the central sealing means 14.

According to the present invention, it is structured that the dialysate is supplied to the blood purification apparatus 15 via both the supply liquid-side pumping chamber P1 and the supply liquid-side sub-pumping chamber P1a and the waste dialysate is discharged from the blood purification apparatus 15 via both the waste liquid-side pumping chamber P2 and the waste liquid-side sub-pumping chamber P2a. More particularly as shown in Fig. 4, the dialysate supplying line L1 is connected between the dialysate inlet port 15c of the blood purification apparatus 15 and the projected connection port 1b and the dialysate discharging line L2 is connected between the dialysate discharging port 15d of the blood purification apparatus 15 and the projected connection port 1c.

In addition, a flow route L4 forming part of the dialysate supplying line L1 is connected between the projected connection port 1a and the supply liquid-side outlet port 4 and the supply liquid-side inlet port 3 is connected to a flow route L3 forming part of the dialysate supplying line L1. The flow route L3 is further connected to the supplying source (not shown) of the dialysate conditioned to a predetermined concentration and thus the dialysate fed from the supplying source can be supplied to the blood purification apparatus 15 through the supply liquid-side pumping chamber P1 and the supply liquid-side sub-pumping chamber P1a.

On the other hand, a flow route L5 forming part of the dialysate discharging line L2 is connected to the projected connection port 1d and the waste liquid-side inlet port 5 and the waste liquid-side outlet port 6 is connected to a flow route L6 forming part of the dialysate discharging line L2. The flow route L6 is connected to any discharged liquid recovering means (not shown) and thus the discharged liquid from the blood purification apparatus 15 can be recovered to the discharged liquid recovering means through the waste liquid-side sub-pumping chamber P2a and the waste liquid-side pumping chamber P2. Reference characters Va, Vb in Fig. 4 denotes back pressure valves connected respectively to the flow route L4, L6.

When the plunger 2 is moved by the motor M toward the right in Fig. 4, the dialysate is sucked into the supply liquid-side pumping chamber P1 from the dialysate supplying source and simultaneously the diaphragm 8 is also pulled and thus deflected toward the right. This reduces the volume of the supply liquid-side sub-pumping chamber P1a and thus the dialysate is supplied to the blood purification apparatus 15 from the supply liquid-side sub-pumping chamber P1a via the dialysate supplying line L1. On the other hand, when the plunger 2 is moved by the motor M toward the right in Fig. 4, the dialysate is supplied to the blood purification apparatus 15 from the supply liquid-side pumping chamber P1 and simultaneously the diaphragm 8 is also pushed and thus deflected toward the left. This increases the volume of the supply liquid-side sub-pumping chamber P1a and thus part of the dialysate supplied to the blood purification apparatus 15 is absorbed or supplemented by the supply liquid-side sub-pumping chamber P1a.

If the discharging capacities of the supply liquid-side pumping chamber P1 and the waste liquid-side pumping chamber P2 are set about twice those of the supply liquid-side sub-pumping chamber P1a and the waste liquid-side sub-pumping chamber P2a, the pulsation caused during the dialysate is supplied to the blood purification apparatus 15 from the reciprocation pump can be reduced. In this case the pulsation will be reduced if the discharging capacities of the supply liquid-side pumping chamber P1 and the waste liquid-side pumping chamber P2 are set larger than those of the supply liquid-side sub-pumping chamber P1a and the waste liquid-side sub-pumping chamber P2a.

Other connecting arrangement between the reciprocation pump and the blood purification apparatus 15 than that shown and described with reference to Fig. 4 can be adopted if the dialysate is supplied to the blood purification apparatus 15 via both the supply liquid-side pumping chamber P1 and the supply liquid-side sub-pumping chamber P1a and the waste dialysate is discharged from the blood purification apparatus 15 via both the waste liquid-side pumping chamber P2 and the waste liquid-side sub-pumping chamber P2a. For example, it is possible to suck the dialysate from the supplying source via the projected connection port 1a and to discharge the waste dialysate via the projected connection port 1d by connecting the supply liquid-side outlet port 4 and the dialysate inlet port 15c as well as the waste liquid-side inlet port 5 and the dialysate outlet port 15d, and also by connecting the supply liquid-side inlet port 3 and the projected connection port 1b as well as the waste liquid-side outlet port 6 and the projected connection port 1c.

According to the reciprocation pump of the preferable embodiment of the present invention, since the supply liquid-side sub-pumping chamber P1a and the waste liquid-side sub-pumping chamber P2a are arranged respectively adjacent to the supply liquid-side pumping chamber P1 and the waste liquid-side pumping chamber P2 at the external side thereof; and sealing means 12, 13 are arranged respectively in walls through which the rod 7 extends for partitioning the supply liquid-side pumping chamber P1 and the supply liquid-side sub-pumping chamber P1a as well as the waste liquid-side pumping chamber P2 and the waste liquid-side sub-pumping chamber P2a, it is possible to prevent the dialysate to be supplied to the blood purification apparatus 15 as well as the waste dialysate from the blood purification apparatus 15 from being leaked to the outside of the reciprocation pump.

In addition, since the reciprocation pump further comprises diaphragms 8, 9 respectively mounted on the distal end and the proximal end of the rod 7 to form the supply liquid-side sub-pumping chamber P1a and the waste liquid-side sub-pumping chamber P2a respectively, it is possible to eliminate necessity of provision of separate sealing means at the mounting portions of the diaphragms. Furthermore, since the dialysate is supplied to the blood purification apparatus 15 through both the supply liquid-side pumping chamber P1 and the supply liquid-side sub-pumping chamber P1a, and the waste dialysate is discharged to the outside through both the waste liquid-side pumping chamber P2 and the waste liquid-side sub-pumping chamber P2a, it is possible to supply leaked dialysate to the blood purification apparatus 15 through the supply liquid-side sub-pumping chamber P1a if the diasylate would be leaked from the supply liquid-side pumping chamber P1 to the supply liquid-side sub-pumping chamber P1a due to aged degradation of the sealing means 12 and similarly possible to discharge the leaked waste dialysate from the waste liquid-side sub-pumping chamber P2a to the outside if the waste dialysate would be leaked from the waste liquid-side pumping chamber P2 to the waste liquid -side sub-pumping chamber P2a.

In addition, since a central sealing means 14 is arranged between the supply liquid-side pumping chamber P1 and the waste liquid-side pumping chamber P2 at a substantially reciprocating center of the plunger 2 to seal the supply liquid-side pumping chamber P1 and the waste liquid-side pumping chamber P2 from each other, the leaked dialysate can flow from the supply liquid-side pumping chamber P1 to the waste liquid-side pumping chamber P2 or the leaked waste dialysate can flow from the waste liquid-side pumping chamber P2 to the supply liquid-side pumping chamber P1, and thus it is possible to prevent the leaked dialysate and waste dialysate from being leaked to the outside of the reciprocation pump.

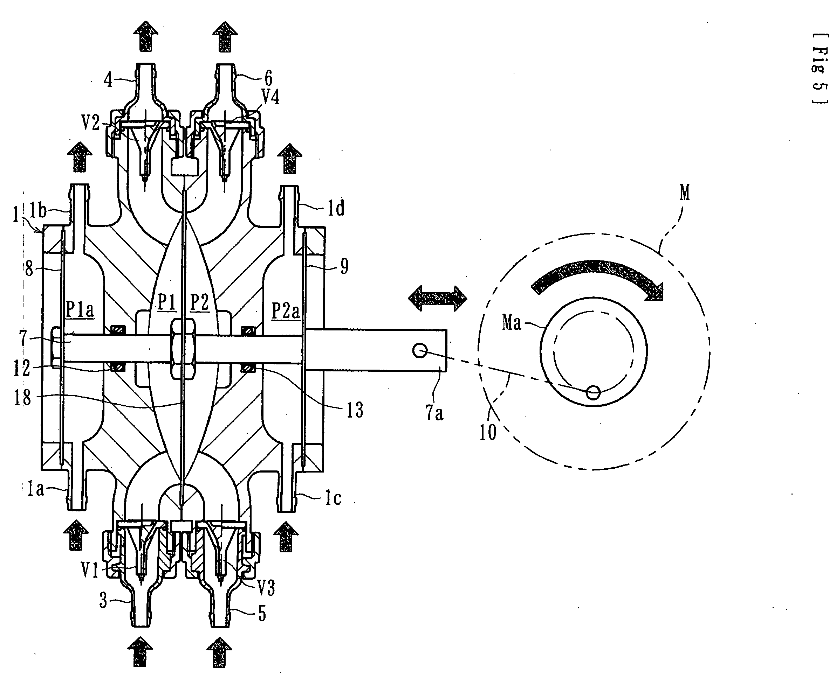

Although the preferable embodiment of the present invention has been described above, the present invention is not limited to this. Fig. 5 shows another embodiment of the present invention which comprises a diaphragm 18 in place of the plunger 2 mounted on the rod 7 for forming the supply liquid-side pumping chamber P1 and the waste liquid side pumping chamber P2. Also in this embodiment, since the supply liquid-side sub-pumping chamber P1a and the waste liquid-side sub-pumping chamber P2a are arranged respectively adjacent to the supply liquid-side pumping chamber P1 and the waste liquid-side pumping chamber P2 at the external side thereof; and sealing means 12, 13 are arranged respectively in walls through which the rod 7 extends for partitioning the supply liquid-side pumping chamber P1 and the supply liquid-side sub-pumping chamber P1a as well as the waste liquid-side pumping chamber P2 and the waste liquid-side sub-pumping chamber P2a, it is possible to prevent the dialysate to be supplied to the blood purification apparatus 15 as well as the waste dialysate from the blood purification apparatus 15 from being leaked to the outside of the reciprocation pump. Furthermore in this embodiment, since the reciprocation means is formed by the diaphragm 18, it is possible to eliminate the central sealing means 14 for sealingly form the supply liquid-side pumping chamber P1 and the waste liquid-side pumping chamber P2 as compared with a case using plunger 2 for this purpose.

In the reciprocation pump of the present invention, the connection arrangement between the reciprocation pump and the blood purification apparatus 15 is not limited to that shown in Fig. 4 and various connection arrangements can be adopted, if the connection arrangement is formed so that the dialysate can be supplied to the blood purification apparatus 15 through both the supply liquid-side pumping chamber P1 and the supply liquid-side sub-pumping chamber P1a as well as the waste dialysate from the blood purification apparatus 15 can be discharged to the outside through both the waste liquid-side pumping chamber P2 and the waste liquid-side sub-pumping chamber P2a. In addition, the sealing means 12, 13 and the central sealing means 14 may be other forms of sealing means than an O-ring.

Furthermore, the present invention is not limited to a reciprocation pump used for supplying dialysate to a blood purification apparatus and can be applied to other kinds of reciprocation pumps (whole industrial reciprocation pumps used for common use) which supply liquids to other objective apparatus than the blood purification apparatus. Although in such a case, it is possible to prevent a liquid to be supplied to the objective apparatus as well as the waste liquid from the objective apparatus from being leaked to the outside of the reciprocation pump, by arranging the supply liquid-side sub-pumping chamber and the waste liquid-side sub-pumping chamber respectively adjacent to the supply liquid-side pumping chamber and the waste liquid-side pumping chamber at the external side thereof; and by arranging sealing means respectively in walls through which the rod extends for partitioning the supply liquid-side pumping chamber and the supply liquid-side sub-pumping chamber as well as the waste liquid-side pumping chamber and the waste liquid-side sub-pumping chamber.

### Applicability in Industry

The present invention can be applied to a reciprocation pump and a dialysis apparatus equipped with such a reciprocation pump having a different external appearance from that shown in the accompanied drawings or having additional functions, if the reciprocation pump comprises a supply liquid-side sub-pumping chamber arranged adjacent to the supply liquid-side pumping chamber at the external side thereof; a waste liquid-side sub-pumping chamber arranged adjacent to the waste liquid-side pumping chamber at the external side thereof; a rod extending through the supply liquid-side sub-pumping chamber, the supply liquid-side pumping chamber, the waste liquid-side pumping chamber and the waste liquid-side sub-pumping chamber and being connected to the reciprocation means and the driving source to transmit a driving force of the driving source to the reciprocation means; and sealing means arranged respectively in walls through which the rod extends for partitioning the supply liquid-side pumping chamber and the supply liquid-side sub-pumping chamber as well as the waste liquid-side pumping chamber and the waste liquid-side sub-pumping chamber.

### Description of Reference Numerals

1 casing
2 plunger (reciprocation means)
3 supply liquid-side inlet port
4 supply liquid-side outlet port
5 waste liquid-side inlet port
6 waste liquid-side outlet port
7 rod
8 diaphragm
9 diaphragm
10 connecting rod
11 crosshead cylinder
12 sealing means
13 sealing means
14 central sealing means
15 blood purification apparatus (objective apparatus)
16 arterial-side blood circuit
17 veneous-side blood circuit
18 diaphragm (reciprocation means)
M motor (driving source)
P1 supply liquid-side pumping chamber
P1a supply liquid-side sub-pumping chamber
P2 waste liquid-side pumping chamber
P2a waste liquid-side sub-pumping chamber

## Claims

1. A reciprocation pump comprising:
a supply liquid-side pumping chamber (P1) for supplying a liquid from a source to an objective apparatus (15);
a waste liquid-side pumping chamber (P2) for discharging a waste liquid discharged from the objective apparatus (15) to the outside;
a reciprocation means (2) being able to reciprocate between the supply liquid-side pumping chamber (P1) and the waste liquid-side pumping chamber (P2), the reciprocating motion of the reciprocation means (2) performing suction and discharge of the liquid to and from the supply liquid-side pumping chamber (P1) as well as suction and discharge of the waste liquid to and from the waste liquid-side pumping chamber (P2); and
a driving source (M) for driving the reciprocation means (2);
the reciprocation pump adapted to supply the liquid from the source to the objective apparatus (15) with the supply liquid-side pumping chamber (P1) and to discharge the waste liquid from the objective apparatus (15) to the outside with the waste liquid-side pumping chamber (P2) and **characterized in** further comprising:
a supply liquid-side sub-pumping chamber (P1a) arranged adjacent to the supply liquid-side pumping chamber (P1) at an external side thereof
a waste liquid-side sub-pumping chamber (P2a) arranged adjacent to the waste liquid-side pumping chamber (P2) at an external side thereof;
a rod (7) extending through the supply liquid-side sub-pumping chamber (P1a), the supply liquid-side pumping chamber (P1), the waste liquid-side pumping chamber (P2) and the waste liquid-side sub-pumping chamber (P2a) and being connected to the reciprocation means (2) and the driving source (M) to transmit a driving force of the driving source (M) to the reciprocation means (2); and
sealing means (12, 13) arranged respectively in walls through which the rod (7) extends for partitioning the supply liquid-side pumping chamber (P1) and the supply liquid-side sub-pumping chamber (P1a) as well as the waste liquid-side pumping chamber (P2) and the waste liquid-side sub-pumping chamber (P2a).

2. A reciprocation pump of claim 1 wherein the reciprocation pump further comprises diaphragms (8, 9) respectively mounted on the distal end and the proximal end of the rod (7) to form the supply liquid-side sub-pumping chamber (P1a) and the waste liquid-side sub-pumping chamber (P2a) respectively.

3. A reciprocation pump of claim 2 wherein the liquid is supplied to the objective apparatus (15) through both the supply liquid-side pumping chamber (P1) and the supply liquid-side sub-pumping chamber (P1a), and wherein waste liquid is discharged to the outside through both the waste liquid-side pumping chamber (P2) and the waste liquid-side sub-pumping chamber (P2a).

4. A reciprocation pump of any one of claims 1~3 wherein the reciprocation means (2) comprises a plunger (2) formed on the rod (7) to form the supply liquid-side pumping chamber (P1) and the waste liquid-side pumping chamber (P2), and wherein a central sealing means (14) is arranged between the supply liquid-side pumping chamber (P1) and the waste liquid-side pumping chamber (P2) at a substantially reciprocating center of the plunger (2) to seal the supply liquid-side pumping chamber (P1) and the waste liquid-side pumping chamber (P2) from each other.

5. A reciprocation pump of any one of claims 1~3 wherein the reciprocation means (2) comprises a diaphragm (18) mounted on the rod (7) to form the supply liquid-side pumping chamber (P1) and the waste liquid-side pumping chamber (P2).

6. A reciprocation pump of any one of claims 1~5 wherein the objective apparatus (15) is a blood purification apparatus and the liquid is dialysate.

7. A dialysis apparatus equipped with the reciprocation pump of claim 6.

## Patentansprüche

1. Hubkolbenpumpe, aufweisend:
eine zufuhrflüssigkeitsseitige Pumpkammer (P1) zum Zuführen einer Flüssigkeit von einer Quelle zu einer Zielvorrichtung (15);
eine ablaufflüssigkeitsseitige Pumpkammer (P2) zum Abführen einer verbrauchten Flüssigkeit, die von der Zielvorrichtung (15) nach außen abgeführt wird;
eine Hin-und-Herbewegungseinrichtung (2), die fähig ist, eine Hin-und-Herbewegung zwischen der zufuhrflüssigkeitsseitigen Pumpkammer (P1) und der ablaufflüssigkeitsseitigen Pumpkammer (P2) zu bewerkstelligen, wobei die Hin-und-Herbewegung der Hin-und-Herbewegungseinrichtung (2) ein Ansaugen und ein Ausstoßen der Flüssigkeit in die bzw. aus der zufuhrflüssigkeitsseitigen Pumpkammer (P1), sowie ein Ansaugen und Ausstoßen der verbrauchten Flüssigkeit in die bzw. aus der ablaufflüssigkeitsseitigen Pumpkammer (P2) durchführt; und
eine Antriebsquelle (M) zum Antreiben der Hin-und-Herbewegungseinrichtung (2);
wobei die Hubkolbenpumpe ausgebildet ist, um mit der zufuhrflüssigkeitsseitigen Pumpkammer (P1) die Flüssigkeit von der Quelle zur Zielvorrichtung (15) zuzuführen und mit der ablaufflüssigkeitsseitigen Pumpkammer (P2) die verbrauchte Flüssigkeit aus der Zielvorrichtung (15) nach außen hin abzuführen, und sie **dadurch gekennzeichnet ist, dass** sie weiter aufweist:
eine zufuhrflüssigkeitsseitige Pumpnebenkammer (P1a), die benachbart zur zufuhrflüssigkeitsseitigen Pumpkammer (P1) an deren Außenseite angeordnet ist,
eine ablaufflüssigkeitsseitige Pumpnebenkammer (P2a), die benachbart zur ablaufflüssigkeitsseitigen Pumpkammer (P2) an deren Außenseite angeordnet ist,
eine Stange (7), die sich durch die zufuhrflüssigkeitsseitige Pumpnebenkammer (P1a), die zufuhrflüssigkeitsseitige Pumpkammer (P1), die ablaufflüssigkeitsseitige Pumpkammer (P2), und die ablaufflüssigkeitsseitige Pumpnebenkammer (P2a) hindurch erstreckt und mit der Hin-und-Herbewegungseinrichtung (2) und der Antriebsquelle (M) verbunden ist, um eine Antriebskraft der Antriebsquelle (M) zur Hin-und-Herbewegungseinrichtung (2) zu übertragen; und
eine Abdichtungseinrichtung (12, 13), die jeweils in Wänden angeordnet ist, durch welche sich die Stange (7) hindurch erstreckt, um die zufuhrflüssigkeitsseitige Pumpkammer (P1) und die zufuhrflüssigkeitsseitige Pumpnebenkammer (P1a) sowie die ablaufflüssigkeitsseitige Pumpkammer (P2) und die ablaufflüssigkeitsseitige Pumpnebenkammer (P2a) voneinander abzutrennen.

2. Hubkolbenpumpe nach Anspruch 1, wobei die Hubkolbenpumpe weiter Membranen (8, 9) aufweist, die jeweils an dem distalen Ende und dem proximalen Ende der Stange (7) angeordnet sind, um die zufuhrflüssigkeitsseitige Pumpnebenkammer (P1a) bzw. die ablaufflüssigkeitsseitige Pumpnebenkammer (P2a) zu bilden.

3. Hubkolbenpumpe nach Anspruch 2, wobei die Flüssigkeit zur Zielvorrichtung (15) durch sowohl die zufuhrflüssigkeitsseitige Pumpkammer (P1) als auch die zufuhrflüssigkeitsseitige Pumpnebenkammer (P1a) zugeführt wird, und wobei verbrauchte Flüssigkeit nach außen durch sowohl die ablaufflüssigkeitsseitige Pumpkammer (P2) als auch die ablaufflüssigkeitsseitige Pumpnebenkammer (P2a) abgeführt wird.

4. Hubkolbenpumpe nach einem der Ansprüche 1 bis 3, wobei die Hin-und-Herbewegungseinrichtung (2) einen Plunger (2) aufweist, der auf der Stange (7) ausgebildet ist, um die zufuhrflüssigkeitsseitige Pumpkammer (P1) und die ablaufflüssigkeitsseitige Pumpkammer (P2) zu bilden, und wobei eine mittlere Abdichtungseinrichtung (14) zwischen der zufuhrflüssigkeitsseitigen Pumpkammer (P1) und der ablaufflüssigkeitsseitigen Pumpkammer (P2) im Wesentlichen an einem Hin-und-Herbewegungsmittelpunkt des Plungers (2) angeordnet ist, um die zufuhrflüssigkeitsseitige Pumpkammer (P1) und die ablaufflüssigkeitsseitige Pumpkammer (P2) gegeneinander abzudichten.

5. Hubkolbenpumpe nach einem der Ansprüche 1 bis 3, wobei die Hin-und-Herbewegungseinrichtung (2) eine an der Stange (7) montierte Membran (18) aufweist, um die zufuhrflüssigkeitsseitige Pumpkammer (P1) und die ablaufflüssigkeitsseitige Pumpkammer (P2) zu bilden.

6. Hubkolbenpumpe nach einem der Ansprüche 1 bis 5, wobei die Zielvorrichtung (15) eine Blutreinigungsvorrichtung ist und die Flüssigkeit ein Dialysat ist.

7. Dialysevorrichtung, die mit der Hubkolbenpumpe nach Anspruch 6 ausgerüstet ist.

## Revendications

1. Pompe alternative comprenant :
une chambre de pompage du côté du liquide d'alimentation (P1) pour amener un liquide d'une source à un appareil objectif (15) ;
une chambre de pompage du côté des déchets liquides (P2) pour décharger un déchet liquide déchargé par l'appareil objectif (15) à l'extérieur ;
un moyen alternatif (2) qui peut effectuer un mouvement de va-et-vient entre la chambre de pompage du côté du liquide d'alimentation (P1) et la chambre de pompage du côté des déchets liquides (P2), le mouvement alternatif du moyen alternatif (2) réalisant l'aspiration et la décharge du liquide vers et par la chambre de pompage du côté du liquide d'alimentation (P1) ainsi que l'aspiration et la décharge du déchet liquide vers et de la chambre de pompage du côté des déchets liquides (P2) ; et
une source d'entraînement (M) pour entraîner le moyen alternatif (2) ;
la pompe alternative étant adaptée pour amener le liquide de la source à l'appareil objectif (15) avec la chambre de pompe du côté du liquide d'alimentation (P1) et pour décharger les déchets liquides de l'appareil objectif (15) à l'extérieur avec la chambre de pompage du côté des déchets liquides (P2) et **caractérisée en ce qu'**elle comprend en outre :
une chambre de pompage auxiliaire du côté du liquide d'alimentation (P1a) agencée de manière adjacente à la chambre de pompage du côté du liquide d'alimentation (P1) au niveau de son côté externe,
une chambre de pompage auxiliaire du côté des déchets liquides (P2a) agencée de manière adjacente à la chambre de pompage du côté des déchets liquides (P2) au niveau de son côté externe ;
une tige (7) s'étendant à travers la chambre de pompage auxiliaire du côté du liquide d'alimentation (P1a), la chambre de pompage du côté du liquide d'alimentation (P1), la chambre de pompage du côté des déchets liquides (P2) et la chambre de pompage auxiliaire du côté des déchets liquides (P2a) et étant raccordée au moyen alternatif (2) et à la source d'entraînement (M) pour transmettre une force d'entraînement de la source d'entraînement (M) au moyen alternatif (2) ; et
des moyens d'étanchéité (12, 13) agencés respectivement dans des parois à travers lesquelles la tige (7) s'étend pour séparer la chambre de pompage du côté du liquide d'alimentation (P1) et la chambre de pompage auxiliaire du côté du liquide d'alimentation (P1a) ainsi que la chambre de pompage du côté des déchets liquides (P2) et la chambre de pompage auxiliaire du côté des déchets liquides (P2a).

2. Pompe alternative selon la revendication 1, dans laquelle la pompe alternative comprend en outre des diaphragmes (8, 9) respectivement montés sur l'extrémité distale et l'extrémité proximale de la tige (7) afin de former la chambre de pompage auxiliaire du côté du liquide d'alimentation (P1a) et la chambre de pompage auxiliaire du côté des déchets liquides (P2a) respectivement.

3. Pompe alternative selon la revendication 2, dans laquelle le liquide est amené à l'appareil objectif (15) à la fois par la chambre de pompage du côté du liquide d'alimentation (P1) et la chambre de pompage auxiliaire du côté du liquide d'alimentation (P1a), et dans laquelle les déchets liquides sont déchargés à l'extérieur à la fois par la chambre de pompage du côté des déchets liquides (P2) et la chambre de pompage auxiliaire du côté des déchets liquides (P2a).

4. Pompe alternative selon l'une quelconque des revendications 1 à 3, dans laquelle le moyen alternatif (2) comprend un piston plongeur (2) formé sur la tige (7) afin de former la chambre de pompage du côté du liquide d'alimentation (P1) et la chambre de pompage du côté des déchets liquides (P2), et dans laquelle un moyen d'étanchéité central (14) est agencé entre la chambre de pompage du côté du liquide d'alimentation (P1) et la chambre de pompage du côté des déchets liquides (P2) au niveau d'un centre sensiblement alternatif du piston plongeur (2) afin de sceller la chambre de pompage du côté du liquide d'alimentation (P1) et la chambre de pompage du côté des déchets liquides (P2) l'une de l'autre.

5. Pompe alternative selon l'une quelconque des revendications 1 à 3, dans laquelle le moyen alternatif (2) comprend un diaphragme (18) monté sur la tige (7) afin de former la chambre de pompage du côté du liquide d'alimentation (P1) et la chambre de pompage du côté des déchets liquides (P2).

6. Pompe alternative selon l'une quelconque des revendications 1 à 5, dans laquelle l'appareil objectif (15) est un appareil de purification de sang et le liquide est un dialysat.

7. Appareil de dialyse équipé de la pompe alternative selon la revendication 6.
